Europäisches Patentamt

European Patent Office

Office européen des brevets

(19)

(11) Publication number: 0 163 429
A2

## (12) EUROPEAN PATENT APPLICATION

(21) Application number: 85303050.0

(22) Date of filing: 30.04.85

(51) Int. Cl.⁴: **C 07 C 1/20**
**B 01 J 29/28**

(30) Priority: 21.05.84 US 612174

(43) Date of publication of application:
04.12.85 Bulletin 85/49

(84) Designated Contracting States:
BE DE FR GB IT NL

(71) Applicant: MOBIL OIL CORPORATION
150 East 42nd Street
New York New York 10017(US)

(72) Inventor: Chu, Cynthia Ting-Wah
283 Westcott Boulevard
Pennington New Jersey 08534(US)

(72) Inventor: Chang, Clarence Dayton
11 Murray Place
Princeton New Jersey 08540(US)

(74) Representative: West, Alan Harry
Mobil Court 3 Clements Inn
London WC2A 2EB(GB)

(54) Methanol conversion using reactivated zeolite catalyst materials.

(57) A method of converting methanol into hydrocarbons comprises contacting methanol under conversion conditions with a substantially deactivated ZSM–5 type catalyst which has been reactivated by contact with an activating agent comprising ammonia and steam. Said reactivation occurs at temperatures from 100 to 1000°C to the extent that at least 10% crystallinity is preserved in the activated product.

F-2832-L

## METHANOL CONVERSION USING REACTIVATED
## ZEOLITE CATALYST MATERIALS

This invention relates to a method for converting methanol into hydrocarbons by contacting methanol with substantially deactivated highly siliceous calcined zeolite catalyst materials which are reactivated by contact with a mixture of ammonia and water at temperatures ranging from 100 to 1000°C.

Zeolitic materials, both natural and synthetic, have been demonstrated in the past to have catalytic properties for various types of hydrocarbon conversions. Certain zeolitic materials are ordered, porous crystalline aluminosilicates having a definite crystalline structure containing a large number of channels. These cavities and channels are precisely uniform in size. Since these dimensions enable the pores to accept molecules of certain dimensions for adsorption while rejecting those of larger dimensions, these materials have come to be known as "molecular sieves" and are utilized in a variety of ways to take advantage of these properties.

Such molecular sieves, both natural and synthetic, include a wide variety of positive ion-containing crystalline aluminosilicates. These aluminosilicates can be described as a rigid three-dimensional framework of $SiO_4$ and $AlO_4$ in which the tetrahedra are cross-linked by the sharing of oxygen atoms whereby the ratio of the total aluminum and silicon atoms to oxygen is 1:2. The electrovalence of the tetrahedra containing aluminum is balanced by the inclusion in the crystal of a cation, for example, an alkali metal or an alkaline earth metal cation. This can be expressed wherein the ratio of aluminum to the number of various cations, such as Ca/2, Sr/2, Na, K or Li is equal to unity. One type of cation may often be exchanged either entirely or partially by another type
of cation utilizing ion exchange techniques in a conventional
manner.  By means of such cation exchange, it has been possible to
vary the properties of a given aluminosilicate by suitable selection
of the cation.  The spaces between the tetrahedra are usually
occupied by molecules of water prior to dehydration.

The crystalline zeolites utilized in the present invention
are members of a class of zeolitic materials which exhibit unusual
properties.  These zeolites have shape-selective properties
intermediate between those exhibited by the small-pore and the
large-pore zeolites.  At room temperatures, these zeolites permit
sorption of normal paraffins and singly methyl-branched paraffins,
such as 3-methylpentane, but exclude the bulkier doubly-branched
hydrocarbons such as 2,2-dimethylbutane.  As catalysts, these
zeolites show a variety of unusual properties: they convert methanol
selectively to high-octane gasoline; they remove wax molecules from
petroleum fractions; and they will synthesize ethylbenzene,
isomerize xylene, and disproportionate toluene.  The enhanced
selectivity and low aging characteristics in these processes are a
result of the intermediate pore size of this class of zeolites.

Although these zeolites have unusually low alumina
contents, i.e. high silica to alumina mole ratios, they are very
active even when the silica to alumina mole ratio exceeds 30.  The
activity is surprising since catalytic activity is generally
attributed to framework aluminum atoms and/or cations associated
with these aluminum atoms.  These zeolites retain their
crystallinity for long periods in spite of the presence of steam at
high temperature which induces irreversible collapse of the
framework of other zeolites, e.g. of the X and A type.  Furthermore,
carbonaceous deposits, when formed, may be removed by burning at
higher than usual temperatures to restore activity.  These zeolites,
used as catalysts, generally have low coke-forming activity and therefore are conducive to long times on stream between
regenerations by burning carbonaceous deposits with
oxygen-containing gas such as air.

An important characteristic of the crystal structure of
this class of zeolites is that it provides a selective constrained
access to and egress from the intracrystalline free space by virtue
of having an effective pore size intermediate between the small pore
Linde A and the large pore Linde X, i.e. the pore windows of the
structure are of about a size such as would be provided by
10-membered rings of silicon atoms interconnected by oxygen atoms.
It is to be understood, of course, that these rings are those formed
by the regular disposition of the tetrahedra making up the anionic
framework of the crystalline zeolite, the oxygen atoms themselves
being bonded to the silicon (or aluminum, etc.) atoms at the centers
of the tetrahedra.

The silica to alumina mole ratio referred to may be
determined by conventional analysis. This ratio is meant to
represent, as closely as possible, the ratio in the rigid anionic
framework of the zeolite crystal and to exclude aluminum in the
binder or in cationic or other form within the channels. Although
zeolites with a silica to alumina mole ratio of at least 12 are
useful, it is preferred in some instances to use zeolites having
substantially higher silica/alumina ratios, e.g. 1600 and above. In
addition, zeolites as otherwise characterized herein but which are
substantially free of aluminum, that is zeolites having silica to
alumina mole ratios of up to infinity, are found to be useful and
even preferable in some instances. Such "high silica" or "highly
siliceous" zeolites are intended to be included within this
description.

The class of zeolites used in the present invention, after
activation, acquire an intracrystalline sorption capacity for normal
hexane which is greater than that for water, i.e. they exhibit
"hydrophobic" properties. This hydrophobic character can be used to
advantage in some applications.

The class of zeolites useful herein have an effective pore size such as to freely sorb normal hexane. In addition, the structure must provide constrained access to larger molecules. It is sometimes possible to judge from a known crystal structure whether such constrained access exists. For example, if the only pore windows in a crystal are formed by 8-membered rings of silicon and aluminum atoms, then access by molecules of larger cross-section than normal hexane is excluded and the zeolite is not of the desired type. Windows of 10-membered rings are preferred, although in some instances excessive puckering of the rings or pore blockage may render these zeolites ineffective.

Although 12-membered rings in theory would not offer sufficient constraint to produce advantageous conversions, it is noted that the puckered 12-ring structure of TMA offretite does show some constrained access. Other 12-ring structures may exist which may be operative for other reasons and, therefore, it is not the present intention to entirely judge the usefulness of a particular zeolite solely from theoretical structural considerations.

Rather than attempt to judge from crystal structure whether or not a zeolite possesses the necessary constrained access to molecules of larger cross-section than normal paraffins, a simple determination of the "Constraint Index" as herein defined may be made by passing continuously a mixture of an equal weight of normal hexane and 3-methylpentane over a sample of zeolite at atmospheric pressure according to the following procedure. A sample of the zeolite, in the form of pellets or extrudate, is crushed to a particle size about that of coarse sand and mounted in a glass tube. Prior to testing, the zeolite is treated with a stream of air at 540°C for at least 15 minutes. The zeolite is then flushed with helium and the temperature is adjusted between 290°C and 510°C to give an overall conversion of between 10% and 60%. The mixture of hydrocarbons is passed at 1 liquid hourly space velocity (i.e., 1 volume of liquid hydrocarbon per volume of zeolite per hour) over the zeolite with a helium dilution to give a helium to (total) hydrocarbon mole ratio of 4:1. After 20 minutes on stream, a sample
of the effluent is taken and analyzed, most conveniently by gas
chromatography, to determine the fraction remaining unchanged for
each of the two hydrocarbons.

While the above experimental procedure will enable one to
achieve the desired overall conversion of 10 to 60% for most zeolite
samples and represents preferred conditions, it may occasionally be
necessary to use somewhat more severe conditions for samples of very
low activity, such as those having an exceptionally high silica to
alumina mole ratio. In those instances, a temperature of up to
about 540°C and a liquid hourly space velocity of less than one,
such as 0.1 or less, can be employed in order to achieve a minimum
total conversion of about 10%.

The "Constraint Index" is calculated as follows:

$$\text{Constraint Index} = \frac{\log_{10} \; (\text{fraction of hexane remaining})}{\log_{10} \; (\text{fraction of 3-methylpentane remaining})}$$

The Constraint Index approximates the ratio of the cracking
rate constraints for the two hydrocarbons. Zeolites suitable for
the present invention are those having a Constraint Index of 1 to
12. Constraint Index (CI) values for some typical materials are:

| Zeolite | C.I. |
|---|---|
| ZSM-4 | 0.5 |
| ZSM-5 | 8.3 |
| ZSM-11 | 8.7 |
| ZSM-12 | 2 |
| ZSM-23 | 9.1 |
| ZSM-35 | 4.5 |
| ZSM-38 | 2 |
| ZSM-48 | 3.4 |
| TMA Offretite | 3.7 |
| Clinoptilolite | 3.4 |
| Beta | 1.2 |
| H-Zeolon (mordenite) | 0.4 |
| REY | 0.4 |
| Amorphous Silica-Alumina | 0.6 |
| Erionite | 38 |

The above-described Constraint Index is an important and even critical definition of those zeolites which are useful in the instant invention. The very nature of this parameter and the recited technique by which it is determined, however, admit of the possibility that a given zeolite can be tested under somewhat different conditions and thereby exhibit different Constraint Indices. Constraint Index seems to vary somewhat with severity of operation (conversion) and the presence or absence of binders. Likewise, other variables such as crystal size of the zeolite, the presence of occluded contaminants, etc., may affect the Constraint Index. Therefore, it will be appreciated that it may be possible to so select test conditions as to establish more than one value in the range of 1 to 12 for the Constraint Index of a particular zeolite. Such a zeolite exhibits the constrained access as herein defined and is to be regarded as having a Constraint Index in the range of 1 to 12. Also contemplated herein as having a Constraint Index in the range of 1 to 12 and therefore within the scope of the defined novel class of highly siliceous zeolites are those zeolites which, when tested under two or more sets of conditions within the above-specified ranges of temperature and conversion, produce a value of the Constraint Index slightly less than 1, e.g. 0.9, or somewhat greater than 12, e.g. 14 or 15, with at least one other value within the range of 1 to 12. Thus, it should be understood that the Constraint Index value as used herein is an inclusive rather than an exclusive value. That is, a crystalline zeolite when identified by any combination of conditions within the testing definition set forth herein as having a Constraint Index in the range of 1 to 12 is intended to be included in the instant novel zeolite definition whether or not the same identical zeolite, when tested under other of the defined conditions, may give a Constraint Index value outside of the range of 1 to 12.

The zeolites employed in the present invention are exemplified by ZSM-5, ZSM-11, ZSM-5/ZSM-11 intermediates, ZSM-12, ZSM-23, ZSM-35, ZSM-38, ZSM-48 and other similar materials.

ZSM-5 and its x-ray diffraction pattern are described in greater detail in U.S. Patents 3,702,886 and Re 29,948.

ZSM-11 and its x-ray diffraction pattern are described in greater detail in U.S. Patent 3,709,979.

ZSM-5/ZSM-11 intermediate compositions and their x-ray diffraction patterns are described in U.S. Patent 4,229,424.

ZSM-12 and its x-ray diffraction pattern are described in U.S. Patent 3,832,449.

ZSM-23 and its x-ray diffraction pattern are described in U.S. Patent 4,076,842.

ZSM-35 and its x-ray diffraction pattern are described in U.S. Patent 4,016,245.

ZSM-38 and its x-ray diffraction pattern are more particularly described in U.S. Patent 4,046,859.

ZSM-48 and its x-ray diffraction pattern are more particularly described in U.S. Patent 4,375,573.

It is to be understood that identification of such crystalline zeolites can be resolved on the basis of their respective X-ray diffraction patterns. It is the crystal structure, as identified by the X-ray diffraction "fingerprint", which establishes the identity of the specific crystalline zeolite material.

The specific zeolites described, when prepared in the presence of organic cations, are substantially catalytically inactive, possibly because the intracrystalline free space is occupied by organic cations from the forming solution. Prior art methods of activating can involve heating the zeolite in an inert atmosphere at 540°C for one hour, for example, followed by base exchange with ammonium salts, followed by calcination at 540°C in air. The presence of organic cations in the forming solution may not be absolutely essential to the formation of this type zeolite;
however, the presence of these cations does appear to favor the
formation of this special class of zeolite. More generally, it has
been known to activate this type catalyst by base exchange with
ammonium salts followed by calcination in air at about 540°C for
from about 15 minutes to about 24 hours.

Natural zeolites may sometimes be converted to zeolite
structures of the class herein identified by various activation
procedures and other treatments such as base exchange, steaming,
alumina extraction and calcination, alone or in combinations.
Natural minerals which may be so treated include ferrierite,
brewsterite, stilbite, dachiardite, epistilbite, heulandite, and
clinoptilolite.

The preferred crystalline zeolites for utilization herein
include ZSM-5, ZSM-11, ZSM-12, ZSM-23, ZSM-35, ZSM-38 and ZSM-48,
with ZSM-5 being particularly preferred.

In a preferred aspect of this invention, the zeolites
hereof are selected as those providing among other things a crystal
framework density, in the dry hydrogen form, of not less than about
1.6 grams per cubic centimeter. It has been found that zeolites
which satisfy all three of the discussed criteria are most desired
for several reasons. When hydrocarbon products or by-products are
catalytically formed, for example, such zeolites tend to maximize
the production of gasoline boiling range hydrocarbon products.
Therefore, the preferred zeolites useful with respect to this
invention are those having a Constraint Index as defined above of
about 1 to about 12, a silica to alumina mole ratio of at least
about 12 and a dried crystal density of not less than about 1.6
grams per cubic centimeter. The dry density for known structures
may be calculated from the number of silicon plus aluminum atoms per
1000 cubic Angstroms, as given, e.g., on Page 19 of the article
ZEOLITE STRUCTURE by W. M. Meier. This paper, the entire contents
of which are incorporated herein by reference, is included in
PROCEEDINGS OF THE CONFERENCE ON MOLECULAR SIEVES, (London, April
1967) published by the Society of Chemical Industry, London, 1968.

When the crystal structure is unknown, the crystal framework density may be determined by classical pycnometer techniques. For example, it may be determined by immersing the dry hydrogen form of the zeolite in an organic solvent which is not sorbed by the crystal. Or, the crystal density may be determined by mercury porosimetry, since mercury will fill the interstices between crystals but will not penetrate the intracrystalline free space.

It is possible that the unusual sustained activity and stability of this special class of zeolites is associated with its high crystal anionic framework density of not less than about 1.6 grams per cubic centimeter. This high density must necessarily be associated with a relatively small amount of free space within the crystal, which might be expected to result in more stable structures. This free space, however, is important as the locus of catalytic activity.

Crystal framework densities of some typical zeolites, including some which are not within the purview of this invention, are:

|  | Void Volume | Framework Density |
|---|---|---|
| Ferrierite | 0.28 cc/cc | 1.76 g/cc |
| Mordenite | .28 | 1.7 |
| ZSM-5, -11 | .29 | 1.79 |
| ZSM-12 | – | 1.8 |
| ZSM-23 | – | 2.0 |
| Dachiardite | .32 | 1.72 |
| L | .32 | 1.61 |
| Clinoptilolite | .34 | 1.71 |
| Laumontite | .34 | 1.77 |
| ZSM-4 (Omega) | .38 | 1.65 |
| Heulandite | .39 | 1.69 |
| P | .41 | 1.57 |
| Offretite | .40 | 1.55 |
| Levynite | .40 | 1.54 |
| Erionite | .35 | 1.51 |
| Gmelinite | .44 | 1.46 |
| Chabazite | .47 | 1.45 |
| A | .5 | 1.3 |
| Y | .48 | 1.27 |

When synthesized in the alkali metal form, the zeolite is conveniently converted to the hydrogen form, generally by intermediate formation of the ammonium form as a result of ammonium ion exchange and calcination of the ammonium form to yield the hydrogen form. In addition to the hydrogen form, other forms of the zeolite wherein the original alkali metal has been reduced to less than about 1.5 percent by weight may be used. Thus, the original alkali metal of the zeolite may be replaced by ion exchange with other suitable metal cations of Groups I through VIII of the Periodic Table, including, by way of example, nickel, copper, zinc, palladium, calcium or rare earth metals.

In spite of the aforementioned attributes of these zeolites, attempts have been made to improve these compositions by further enhancing their hydrocarbon conversion activity. Such enhancement is desirable in order to improve the efficiency of catalysts in hydrocarbon conversion process and is of particular importance where shape selective catalysts are concerned. Shape selective catalysts are extremely useful in such processes as xylene isomerization, toluene disproportionation and p-ethyltoluene synthesis which require relatively high para-selectivity. Enhanced shape-selectivity of a catalyst composition can also improve the efficiency of catalytic dewaxing, as well as petrochemical processes. Efforts to enhance the shape-selectivity of such materials in sorptive and catalytic applications by calcining at temperatures of 600 to 1300°C have generally met with disappointing results. While such treated catalyst materials may exhibit enhanced shape-selectivity, they suffer a debilitating loss in catalytic activity. Exposure of zeolites to high temperatures results in a breakdown of crystalline structure and a corresponding loss in activity. Furthermore, zeolites which are calcined to an activity of about zero, generally prove extremely difficult, if not impossible, to reactivate.

Ordinary methods for reactivating these shape-selectivity enhanced zeolite compositions, such as by ammonium ion exchange, have been ineffective. In U.S. Patent 4,324,696, a method is described for preparing superactive synthetic crystalline zeolite catalyst materials which have a high hydrocarbon conversion activity. The catalyst materials are contacted with steam and then base-exchanged with ammonium or acid solution. Data submitted in that application show that steaming alone reduces the activity level while steaming and base-exchange together raise the activity levels many times over that of the starting materials.

U.S. Patent 3,257,310 describes a method of preparing a cracking catalyst of high activity and selectivity by subjecting a crystalline aluminosilicate having pore openings of 6-15A to steam treating at a temperature between about 200 to about 954°C. However, no mention is made of contacting the catalyst with ammonia.

U.S. Patent 4,326,994 relates to a method for increasing the catalytic activity of an acid zeolite having a determinable initial activity which comprises contacting the zeolite with water or steam for a sufficient treating time, temperature and water partial pressure in accordance with the following relationship:

$$0.01(Pt)_T \quad (Pt) \quad 10(Pt)_T \text{ where } (Pt)_T = 2.6 \times 10^{-9} e^{16000/T}$$

where P = water partial pressure (Atm), t = treating time (hours) and T = temperature (°K). Ammonia may be added to the water or steam in amounts ranging from between about 0.01 to about 1.0 mole ratio ammonia to water. However, no mention is made of using this process on substantially deactivated catalysts which have been calcined.

European Patent Application 0 134 330 (published 20 March 1985) describes a process for enhancing the hydrocarbon conversion activity of zeolite materials which are substantially deactivated. Such catalysts are activated by exposure to an activating agent comprising a mixture of steam and ammonia, at temperatures ranging from about 100 to 1000°C. Calcining and activation of the zeolite material is allowed to proceed to the extent that the resulting product retains a crystallinity of at least about 10% as determined by conventional X-ray diffraction studies.

The activity of the resulting catalyst can be measured in terms of its alpha value. The alpha value reflects the relative activity of the catalyst with respect to a high activity silica-alumina cracking catalyst. To determine the alpha value, n-hexane conversion is determined at a suitable temperature between about 288 to 538°C preferably at 538°C. Conversion is varied by variation in space velocity such that a conversion level of up to about 60 percent of n-hexane is obtained and converted to a rate constant per unit volume of zeolite and compared with that of silica-alumina catalyst which is normalized to a reference activity of 538°C . The catalytic activity of the catalyst is then expressed as a multiple of this standard, i.e., the silica-alumina standard. The silica-alumina reference catalyst contains about 10 weight percent $Al_2O_3$ and the remainder $SiO_2$. This method of determining alpha, modified as described above, is described in the Journal of Catalysis, Vol. VI, pages 278-287, 1966, to which reference is made for further details of the method. The extent of the activation produced by the method of the co-pending application is notable. Significant increases in the alpha value may be obtained where such zeolites are activated according to this method.

The activation process employed in Assignee's co-pending application may be used to produce a catalyst material of enhanced shape-selectivity having relatively high hydrocarbon conversion activity. The zeolites are first calcined at temperatures ranging from about 700 to 1300°C, say about 1000°C, in order to enhance their shape selectivity. The resulting materials suffer a loss of activity on calcination in some cases as low as about 0 to 3.5, say less than about 1. The deactivated zeolite can then be activated by exposing the calcined compositions to a mixture of steam and ammonia at temperatures of about 100 to 1000°C. The calcining and activation steps are carried out to the extent that at least about 10% crystallinity is preserved in the resulting product. The molar ratio of steam to ammonia employed can range from about 0.01 to 10, preferably about 1.

The zeolite crystals prepared therein are shaped in a wide variety of particle sizes. Generally speaking, the particles can be in the form of a powder, a granule, or a molded product, such as an extrudate having particle size sufficient to pass through a 2 mesh (Tyler) screen and be retained by a 400 mesh (Tyler) screen. In cases where the catalyst is molded, such as by extrusion, the catalyst crystals can be extruded before drying or dried or partially dried and then extruded.

The resulting zeolites can also be used as a catalyst in intimate combination with a hydrogenating component such as tungsten, vanadium, molybdenum, rhenium, nickel, cobalt, chromium, manganese, or a noble metal such as platinum or palladium where a hydrogenation-dehydrogenation function is to be performed. Such component can be exchanged into the composition, impregnated therein or physically intimately admixed therewith. The hydrogenating component can be impregnated in or on to the zeolite, such as, for example, by, in the case of platinum, treating the zeolite with a solution containing a platinum metal-containing ion. Thus, suitable platinum compounds include chloroplatinic acid, platinous chloride and various compounds containing the tetramineplatinum complex.

The resulting zeolite compositions can be used as catalysts in a number of important reactions. In particular, the subject shape-selectivity enhanced catalysts can be advantageously utilized in xylene isomerization, toluene disproportionation and p-ethyltoluene synthesis processes where high para-selectivity is desirable. The enhanced shape-selectivity of these compositions also commends their use in processes such as catalytic dewaxing. These compositions can also be used as supports for metals in dual-functional catalytic applications such as syngas reactions.

It has now been found that zeolite catalysts prepared from a zeolite which has been shape-selectivity enhanced and substantially deactivated by calcining at temperatures ranging from about 700 to 1300°C, and thereafter activated by contact with an activating agent comprising ammonia and steam at elevated temperatures, are useful as methanol conversion catalysts.

Conventional methods of methanol conversion which employ superatmospheric pressures often produce products of unacceptable durene (1,2,4,5-tetramethylbenzene) content. Durene is a particularly undesirable component of gasoline because of its high melting point (79°C) and its tendency to crystallize out of solution under normal gasoline storage conditions. The process of the present invention is especially useful in preparing hydrocarbons in the gasoline boiling range of low durene content or olefins of low durene content. Methanol to hydrocarbon processes include processes wherein methanol is converted to a hydrocarbon mixture containing gasoline-type hydrocarbons as well as those wherein methanol is converted to a hydrocarbon mixture which contains olefins. Such processes are described in U.S. Patents 3,894,103, 3,894,104, 3,894,107, 4,013,732 and 4,052,479, all of which are incorporated herein by reference. Methanol conversion conditions which are suitable to the present invention include temperatures ranging from about 316 to 649°C, preferably ranging from about 427 to 593°C, pressures ranging from about 100 to 14,000 kPa, preferably ranging from about 100 to 1,480 kPa, and an LHSV ranging from about 0.5 to 500, preferably about 1 to 50, say about 1.

The following Examples illustrate the invention.

## EXAMPLES 1-14
### Preparation of Calcined HZSM-5 Zeolite

HZSM-5 samples having a silica to alumina molar ratio of about 70 were subjected to thermal treatment in ambient atmosphere under varying temperature conditions. Following such treatment, each sample was tested for n-hexane sorption and alpha value. Various samples were also tested for 3-methylpentane sorption, cyclohexane sorption, and crystallinity. HZSM-5 retained good crystallinity for periods in excess of one hour at temperatures below 1000°C. Above 1000°C the rate of framework collapse increased significantly. High temperature-treated HZSM-5 samples were crystalline by X-ray and sorbed 7-12% n-hexane at 25°C after 20 minutes. The zeolites exhibited increased shape-selectivity as calcination temperatures increased. The HZSM-5 zeolites decreased in cracking activity from an alpha value of about 150 for parent materials to an alpha vaue of about 0 for material calcined at 1000°C for one hour. Additional data relating to the thermal treatment is set out in Table I.

## EXAMPLE 15
### Reactivation of Calcined HZSM-5 Zeolites
### by Contact with Ammonia and Steam

The catalyst of Example 5 was used to convert phenol into aniline by ammoniation. Phenol was reacted with excess $NH_3$ (0.5 WHSV) at 510°C, and 2840 kPa. Steam was produced in situ during the reaction. Conversion of phenol was 94.4%. Selectivity to aniline was 90.2%. The catalyst became activated in situ by exposure to steam and ammonia, with the alpha increasing from 0.3 to 3.5. In a control run, ZSM-5 with $SiO_2/Al_2O_3 \sim 26{,}000$ and alpha = 0.3 converted only 1.5% phenol under the same conditions.

## TABLE I

### Effects of Thermal Treatments
### on ZSM-5 Zeolites

| | | | Sorption % | | | | X-ray |
|---|---|---|---|---|---|---|---|
| Example | Treatment | Atmosphere | n-hexane | 3-methyl-pentane | cyclo-hexane | | crystallinity % |
| | Parent HZSM-5 | ambient air | 11.6 | 8.9 | 8.7 | 192 | 100 |
| 1 | 1 hr @ 600°C | " | 11.6 | | | 113-175 | 90 |
| 2 | 1 hr @ 700°C | " | 11.2 | | | 35 | 90 |
| 3 | 1 hr @ 800°C | " | 10.7 | | | 12 (CI=1.4) | 82 |
| 4 | 1 hr @ 900°C | " | 9.8 | | | 4 (CI=0.4) | |
| 5 | 1 hr @ 1000°C | " | 8.3 | 5.4 | slow | 0 | 61 |
| 6 | 1 hr @ 1100°C | | | | | | 25 |
| 7 | 2 hrs @ 1100°C | " | 0 | | | 0 | 0 |
| 8 | 5 min.@ 1000°C | " | 10.2 | | | 0.1 | |
| 9 | 10 min.@ 1000°C | " | 10.0 | | | | |
| 10 | 15 min.@ 1000°C | " | 9.0 | | | | |
| 11 | 3.5 min.@ 1100°C | " | 7.5 | | | | |
| 12 | 10 min.@ 1100°C | " | 3.7 | | | | |
| 13 | 7.5 min.@ 1200°C | " | 4.1 | | | 0 | |
| 14 | 5 min.@ 1200°C | " | 0 | | | 0 | |

## EXAMPLE 16

### Comparison of Selectivity of Reactivated Catalyst
### With Conventional Catalyst in Methanol-to-
### Hydrocarbon Conversion

A reactivated catalyst of the invention was prepared by regenerating the catalyst of Example 15 by air circulation at 538°C resulting in a material having an alpha value of about 3.5. A conventional non-activated HZSM-5 catalyst was also prepared and calcined to a similar alpha value, about 4.0. Methanol was reacted over both catalysts at 500°C, 1 LHSV and 100 kPa for about 3 hours. A comparison of the hydrocarbon products distribution of the respective runs set out in Table II shows a significant difference in hydrocarbon products distribution. For example, the activated calcined catalyst of the present invention shows a much higher (M+P)/O xylenes ratio than the conventional ZSM-5 (8.4 versus 3.3, respectively).

## TABLE II

### Product Distribution of Methanol Conversion

| | Deactivated Calcined HZSM-5 ( = 3.5) (weight percent) | Conventional HZSM-5 ( = 4.0) (weight percent) |
|---|---|---|
| $H_2O$ | 54.74 | 55.94 |
| Dimethyl Ether | 1.21 | 0.33 |
| Methanol | 2.89 | 0.00 |
| CO | 0.40 | 0.25 |
| $CO_2$ | 0.08 | 0.19 |
| $H_2$ | 0.11 | 0.17 |
| Hydrocarbons | 40.57 | 43.09 |
| **Hydrocarbon Distribution** | | |
| Methane | 4.25 | 2.56 |
| Ethane | 0.26 | 0.31 |
| Ethylene | 9.99 | 11.21 |
| Propane | 1.95 | 1.25 |
| Propylene | 31.62 | 38.01 |
| Isobutane | 3.11 | 0.95 |
| n-Butane | 0.68 | 0.39 |
| Butenes | 15.38 | 23.79 |
| Isopentane | 2.60 | 0.73 |
| n-Pentanes | 0.39 | 0.41 |
| Pentenes | 7.98 | 6.81 |
| $C_6$+Aliphatics | 12.31 | 5.21 |
| Benzene | 0.07 | 0.21 |
| Toluene | 1.04 | 1.26 |
| Ethylbenzene | 0.29 | 0.00 |
| M- and p-Xylenes | 3.79 | 2.51 |
| O-Xylene | 0.45 | 0.76 |
| Ethylmethylbenzenes | 1.14 | 0.55 |
| 1,2,4-Trimethylbenzene | 1.35 | 2.18 |
| 1,2,3-Trimethylbenzene | 0.01 | 0.14 |
| Diethylbenzene | 0.37 | 0.10 |
| Durene | 0.38 | 0.41 |
| $C_{10+}$ Aromatics | 0.59 | 0.28 |
| $C_1$ to $C_5$ alkanes | 13.24 | 6.59 |
| $C_2$ to $C_5$ olefins | 64.98 | 79.81 |
| $C_6$+ aliphatics | 12.31 | 5.21 |
| Aromatics | 9.47 | 8.39 |
| Hydrogen/Carbon | 4.18 | 4.07 |
| Oxygen/Carbon | 1.04 | 1.01 |
| Methanol Conversion | 95.42 | 99.53 |

CLAIMS:

1. A method for converting methanol into hydrocarbons which comprises contacting methanol under conversion conditions with a catalyst comprising a highly siliceous crystalline zeolite, having a silica to alumina mole ratio of at least 12 and a constraint index of 1 to 12, the zeolite having been substantially deactivated by calcination at a temperature from 700 to 1300°C and subsequently activated by contact with an activating agent comprising ammonia and steam at a temperature from 100 to 1000°C to the extent that at least 10% crystallinity is preserved in the activated zeolite.

2. A method according to claim 1, wherein the zeolite is selected from ZSM-5, ZSM-11, ZSM-5/ZSM-11 intermediates, ZSM-12, ZSM-23, ZSM-35, ZSM-38 and ZSM-48.

3. A method according to claim 2, wherein the zeolite is in the hydrogen form.

4. A method according to any one of claims 1 to 3, wherein the molar ratio of ammonia to steam is from 0.01 to 10.

5. A method according to any one of claims 1 to 4, wherein the conversion conditions comprise a temperature from 316 to 649°C, a pressure from 100 to 14,000 kPa, and a liquid hourly space velocity from 1 to 50.

6. A method according to claim 5, wherein the conversion conditions comprise a temperature from 427 to 593°C, a pressure from 100 to 1480 kPa and a liquid hourly space velocity from 1 to 50.